# EUROPÄISCHE PATENTSCHRIFT

(11) **EP 2 908 875 B1**
(45) Veröffentlichungstag und Bekanntmachung des Hinweises auf die Patenterteilung: **20.09.2017**
(21) Anmeldenummer: 13783509.6
(22) Anmeldetag: 18.10.2013
(51) Int. Cl.: A61L 27/18, A61L 27/30, A61L 27/32, A61L 27/56, C08L 71/00

(54) **OSTEOKONDUKTIVE BESCHICHTUNG VON KUNSTSTOFFIMPLANTATEN**
OSTEOCONDUCTIVE COATING OF IMPLANTS MADE OF PLASTIC
REVÊTEMENT OSTÉOCONDUCTEUR POUR IMPLANTS EN MATIÈRE PLASTIQUE

(30) Priorität: 19.10.2012 DE 102012020603
(43) Veröffentlichungstag der Anmeldung: 26.08.2015
(73) Patentinhaber: Gerber, Thomas, 18059 Sildemow (DE)
(72) Erfinder: KEUER, Holger, 18057 Rostock (DE)
(74) Vertreter: Uexküll & Stolberg
(86) Internationale Anmeldenummer: PCT/EP2013/071878
(87) Internationale Veröffentlichungsnummer: WO 2014/060591

(56) Entgegenhaltungen:
- EP-A1- 2 238 992
- EP-B1- 1 624 904
- DE-A1-102006 037 497
- DE-A1-102008 044 951
- US-A1- 2009 276 053
- US-B1- 6 602 293

## Beschreibung

Die Erfindung betrifft Verfahren zur Herstellung von Biomaterialien auf der Basis von Kunststoffen wie Polyaryl Polyetherketon (PEK). Im folgenden wird beschrieben, wie eine mechanisch stabile Beschichtung auf Polyaryl Polyetherketon (PEK), z.B. Polyetheretherketon (PEEK), mit einem porösen Knochenersatzmaterial wie z.B. NanoBone® erfolgt, wodurch das Problem der schlechten Zelladhäsion auf derartigen Kunststoff-Oberflächen gelöst werden kann. Das Auftragen des Knochenersatzmaterials kann sowohl trocken als Pulver als auch in einem Nasssprühverfahren erfolgen. Die Beschichtung beruht auf einem kurzzeitigen Aufschmelzen der Polymeroberfläche und dem dadurch teilweise Versinken der zuvor aufgetragenen Schicht. Dabei dringt das geschmolzene Polymer in Nanoporen des Knochenersatzmaterials ein und stellt so eine feste Verbindung her.

Biomaterialien auf der Basis z.B. von Polyetheretherketon (PEEK) haben in den letzten 30 Jahren eine hohe Bedeutung für Implantate in der Unfallchirurgie, der Orthopädie und insbesondere in der Wirbelsäulenchirurgie erlangt. Verantwortlich hierfür sind die hervorragenden mechanischen Eigenschaften und die gute Biokompatibilität.

Einschränkend ist die begrenzte Fixation von PEEK im Kochen, d.h. die PEEK Oberfläche geht mit dem Knochen eine schlechte Verbindung ein ("PEEK Biomaterials in Trauma, Orthopedic, and Spinal Implants", S.M. Kutz and J.N. Devine; Biomaterials, 2007November, 28(32):4845-4869).

Eine Vielzahl von Bemühungen waren daher darauf gerichtet, die schlechte Zell-PEEK Adhäsion zu umgehen und somit den Kochen-Implantat-Übergang zu verbessern.

Hierzu wurden Komposite aus PEEK und Kalziumphosphatbiomaterialien, wie Hydroxylapatit (HA) und beta-Trikalziumphosphat (ß-TCP) entwickelt. Nachteile dieser Komposite sind ihre schlechteren mechanischen Eigenschaften und das Phänomen, dass das keramische Biomaterial und der Kunststoff PEEK keine Verbindung eingehen ("Tensiontension fatigue behavior of hydroxyapatite reinforced polyetheretherketone composites " S.M. Tang et al. Int J Fatigue 2004;26;49-57).

Ein anderer Weg zur Verbesserung des Knochen-Implantat-Kontaktes wurde durch verschiedene Beschichtungen beschritten (zum Beispiel: Cook SD, Rust-Dawicki AM. Preliminary evaluation of titanium-coated PEEK dental implants. Journal of oral implantology 1995;21(3):176-181; Ha S, Mayer J, Koch B, Wintermantel E. Plasma-sprayed hydroxylapatite coating on carbon fibre reinforced thermoplastic composite materials. J Mat Sei 1994;5:481-484).

Auch chemische Modifikationen der PEEK Oberfläche wurden entwickelt (zum Beispiel: Noiset O, Schneider YJ, Marchand-Brynaert J. Adhesion and growth of CaCo2 cells on surfacemodified PEEK substrata. Journal of biomaterials science Polymer edition 2002;11(7):767-786 und Noiset O, Schneider YJ, Marchand-Brynaert J. Fibronectin adsorption or/and covalent grafting on chemically modified PEEK film surfaces. J Biomater Sci Polym Ed 1999;10(6):657-677).

Bekannt sind PEEK Implantate mit Calciumphosphat (S.-W. Ha et al.; Surface activation of polyetheretherketone (PEEK) and formation of calcium phosphate coatings by precipitation. JOURNAL OF MATERIALS SCIENCE MAERIALS IN MEDICINE 8 (1997) 683-69 0; EP 2238992: biologisches Implantat mit geschäumter Oberfläche; US 6602293: orthopädisches Implantat aus thermoplastischen Polymer mit poröser Beschichtung). oder Titan Plasmaspray-beschichteten Oberflächen (C.-M. Han et al.; The electron beam deposition of titanium on polyetheretherketone (PEEK) and the resulting enhanced biological properties. BIOMATERIALS 31 (2010) 3465-3470) bzw. einer Titanbeschichtung mit anschließender Calciumphosphat-Beschichtung (S.-W. Ha et al.; Topological characterization and microstructural interface analysis of vacuum-plasma-sprayed titanium and hydroxyapatite coatings on carbon fibre-reinforced poly(etheretherketone). JOURNAL OF MATERIALS SCIENCE MAERIALS IN MEDICINE 8 (1997) 891-8 96), oder PEEK Implantate mit Hydroxyapatit beschichtet durch ein Plasmaspray-Verfahren (US 2009/0276053). Nachteil der durch Plasmaspray hergestellten Schichten ist die Tatsache, dass diese Schichten relativ dick sind und die Gefahr besteht, dass sie durch mechanische Einwirkungen z. B. beim Einbringen des Implantates abplatzen.

Zum Beispiel zeigt S.-W. Ha, dass die Adhäsion zwischen PEEK und HA Schicht nur sehr gering ist (S.-W. Ha et al.; Plasma-sprayed hydroxylapatite coating on carbon fibre reinforced thermoplastic composite materials. JOURNAL OF MATERIALS SCIENCE MAERIALS IN MEDICINE 5 (1994) 481-484).

Dieser Abrieb der Implantatschicht wirkt sich negativ auf das umliegende Gewebe aus. Insbesondere metallische Titanpartikel zeigen negative Auswirkungen. Die positiven bioaktiven Eigenschaften des Titans sind letztendlich durch die Oxidschicht auf der Oberfläche bedingt. Des Weiteren hat ein Abplatzen oder ein Abbau der aufgetragenen Schicht wieder eine schlechte Zell-PEEK Kontaktfläche zur Folge.

Aufgabe der Erfindung ist es, die Oberfläche von PEK Implantaten so zu modifizieren, dass ein gutes Interface zwischen Knochen und Implantat entsteht. Diese Modifikation soll weder die mechanischen Eigenschaften des Implantates beeinträchtigen noch soll eine *in vivo* Anwendung z. B. durch abplatzende Oberflächenbereiche beeinflusst werden.

Zur Lösung dieser Aufgabe hilft folgende überraschende Beobachtung. Wird ein Granulat eines hochporösen Silicagels, in das HA eingebettet ist, auf eine PEEK Oberfläche gelegt und wird die Oberfläche zum Schmelzen gebracht, dringt das flüssige PEEK in die Nanoporen des Kieselgels und das Granulat versinkt teilweise in dem aufgeschmolzenen PEEK. Ein Kunststoffimplantat wird beschrieben, bei dem in dem Bereich bzw. den Bereichen, in denen Knochen an das Implantat anwachsen soll, in der Oberflächeschicht ein hochporöses Knochenersatzmaterial im Kunststoff eingebettet ist, wobei das Knochenersatzmaterial aus der Oberfläche herausragt. Dabei liegt die Porosität des Knochenersatzmaterials bevorzugt im Bereich von 20 bis 80%, und/oder die mittlere Porengröße (des Knochenersatzmaterials) bevorzugt im Bereich von 10 bis 100 nm. Dabei hat die Oberflächenschicht des Kunststoffes bevorzugt eine Stärke von 1µm bis 100µm und/oder das Knochenersatzmaterial ragt bevorzugt im Bereich von 0,01 µm - 50 µm, mehr bevorzugt im Bereich von 0.1 bis 30µm oder im Bereich von 1 bis 20 µm aus dem Kunststoff heraus.

Bevorzugt ist das Knochenersatzmaterial kristallines Hydroxylapatit (HA), eingebettet in einer amorphen porösen Matrix aus Siliziumdioxid, wobei das HA im Bezug auf HA und Siliziumdioxid eine Anteil von 20 bis 90 Gewichtsprozent hat, es als Granulat mit einer Größe bevorzugt im Bereich von 1 bis 50µm vorliegt oder eine kontinuierliche Schicht mit einer bevorzugten Schichtdicke von 1 bis 20µm ist.

Als Granulat wurde ein Knochenersatzmaterial verwendet, das durch das Patent EP 1624904B1 beschrieben wird. EP 1624904B1 offenbart ein Granulat auf Basis von Calciumphosphat, bei dem kristallines Calciumphosphat in eine Siliziumdioxid-Xerogelmatrix eingebettet ist. Dies ist erhältlich durch Herstellung des Calciumphosphats über eine Fällungsreaktion, bei der man die Lösung mit dem ausgefällten Calciumphosphat z.B. durch Rühren homogenisiert, man eine hochkonzentrierte Kieselsäurelösung zugibt, man das Gemisch durch die anschließend einsetzende Gelbildung fixiert und man es durch Entfernen des Lösungsmittels in eine Xerogelmatrix überführt. Dabei weisen die in der Xerogelmatrix liegenden Calciumphosphat-Kristallite eine Größe von etwa 10 nm bis etwa 2000 nm und die Granulatkörner eine Größe von etwa 1 µm bis etwa 1000 µm auf, und der Siliziumdioxid-Anteil liegt im Bereich von etwa 2 bis etwa 80 Gew.-%, vorzugsweise im Bereich von etwa 4 bis etwa 50 Gew.-%, bezogen auf die Gesamtmasse der Granulatkörner. EP 1624904B1 offenbart auch ein Material mit Granulatkörnern, welche individuelle Hydroxylapatit-Kristallite umfassen, die in eine Siliziumdioxid-Xerogel-Matrix eingebettet und davon umschlossen sind, wobei die individuellen Hydroxylapatit-Kristallite eine Größe von ca. 10 nm bis ca. 2000 nm aufweisen und homogen in der Matrix verteilt sind, wobei die Granulatkörner eine Größe von ca. 1 µm bis ca. 1000 µm aufweisen, und wobei das Siliziumdioxid etwa 2 bis etwa 80 Gewichtsprozent der Gesamtmasse der Granulatkörner ausmacht. In EP 1624904B1 wird auch ein Knochenersatzmaterial offenbart, welches Granulatkörner dieses Granulats umfasst, die eine dreidimensionale Struktur bilden, die neben den in den Granulatkörnern vorhandenen Poren ferner Poren etwa in der Größe der Granulatkörner aufweist. Damit ist das Knochenersatzmaterial hoch porös.

Derartige Knochenersatzmaterialien sind u.a. als NanoBone® kommerziell erhältlich (Artoss GmbH, Rostock, Deutschland). Es können jedoch auch andere poröse Knochenersatzmaterialien verwendet werden, z.B. Bio-Oss® der Geistlich Pharma AG, Deutschland. Bevorzugt weisen die Knochenersatzmaterialien eine hohe Porosität auf (bevorzugt ca. 10% bis ca. 90%, insbesondere ca. 20% bis ca. 80% oder ca. 40% bis ca. 80%, ca. 50% bis ca. 70%). Porosität kann z.B. mit dem Standardverfahren der Quecksilberporosimetrie gemessen werden.

Das verwendete Material hat eine innere Oberfläche von ca 200 m²/g (gemessen mit Gasadsorbtion (BET). Die Porosität betrug ca. 60%, wobei die Porengröße einigen Nanometer bis 20 nm dominiert.

Bevorzugt ist der Kunststoff Polyetheretherketon (PEEK), es kann aber auch z.B. Polyaryl Polyetherketon (PEK), insbesondere Polyetherketonketon (PEKK) verwendet werden.

In dem erfindungsgemäßen Implantat ist der Kunststoff in Nanoporen des Knochenersatzmaterials eingedrungen. Insbesondere sind Nanoporen des Knochenersatzmaterials in der Oberflächenschicht des Kunststoffs mit diesem gefüllt, so dass im Übergang vom Kunststoffimplantat zu Knochenersatzmaterial (in der Oberflächenschicht) ein Verbund aus Kunststoff und Knochenersatzmaterial von mehreren Mikrometern Dicke entsteht.

Gegenstand der Erfindung ist ein Verfahren zur Herstellung eines erfindungsgemäßen Kunststoffimplantats, bei dem eine Form, z.B. die Form zur Herstellung des Implantates, an den Stellen, an denen das Implantat Knochenkontakt haben soll (bzw. an denen Knochen an das Implantat anwachsen soll), mit einem wässrigen Schlicker aus einem hochporösem Knochenersatzmaterial (wie oben beschrieben) beschichtet wird, die Schicht getrocknet und anschließend der plastifizierte Kunststoff in die Form gegeben wird. Dann erhitzt man bzw. bringt die Oberfläche des Kunststoffs zum Schmelzen. Dazu kann z.B. Induktions-Erhitzen der metallischen Form für das Implantat bis zu 300-500°C, bevorzugt 380-400°C, gemessen an der Oberfläche der Form, für ca. 3 - ca. 20 Sekunden, bevorzugt 5-10 Sekunden, angewandt werden (z.B. mit TIG 30/100, HÜTTINGER Elektronik GmbH). Auch ein heißer Luftstrom kann, z.B. bei einer nicht-metallischen Form, zum Erhitzen verwendet werden.

Bevorzugt besteht der Schlicker aus Wasser und einem Granulat von kristallinem Hydroxylapatit (HA), eingebettet in einer amorphen porösen Matrix aus Siliziumdioxid, wobei die Granulatgröße bevorzugt im Bereich von etwa 1 bis 50 µm oder etwa 5 bis 10 µm, z.B. kleiner als 10 µm, liegt.

Mit dem beobachteten Effekt lässt sich ein ganzer Formkörper beschichten. Wird das beschriebene Knochenersatzmaterial gemahlen und gesiebt, so dass Granulatkörner bevorzugt kleiner 10µm verbleiben, und wird daraus mit Wasser ein Schlicker erzeugt, kann eine Gussform eingestrichen und getrocknet werden. Wird anschließend der plastifizierte Kunststoff in die Form gegeben, entsteht der oben beschrieben Effekt. Der Kunststoff dringt in die Nanoporen ein. Es entsteht in einer Oberflächenschicht ein Verbund aus Kunststoff und Silicagel/HA. An der Oberfläche ist letztendlich das Silica/HA Material dominierend.

Wie Beispiel 1 und 2 zeigen, wird dadurch ein perfekter Kontakt von PEEK Implantat und Knochen erreicht.

Ein zweites Verfahren kann z.B. genutzt werden, wenn die Implantatformgebung unabhängig von der Oberflächenmodifikation sein soll. Es können also sowohl rotationssymmetriche als auch nicht rotationssymmetrische Implantate beschichtet werden. In diesem Fall muss nicht auf ein vorher produziertes Knochenersatzmaterial zurückgegriffen werden.

Kern dieses Verfahrens ist ein Sol-Gel Beschichtungsprozess.

Gegenstand der Erfindung ist damit auch ein Verfahren zur Herstellung eines erfindungsgemäßen Kunststoffimplantats, bei dem
- die Bereiche des Implantates, an denen Knochen anwachsen soll, hydrophilisiert werden, zum Beispiel durch ein Sauerstoffplasma, wobei z.B. eine Niederdruckplasmakammer oder ein Plasmajet unter Normaldruck genutzt werden kann,
- ein Siliziumdioxidsol, in das kristallines Hydroxylapatit dispergiert wird bzw. dispergiert ist, wobei die Feststoffkonzentration (HA und SiO₂ des Sols im Bereich von 0.2 bis 10 Gew.-% liegt und das Verhältnis von HA zu SiO₂ im Bereich von 90 zu 10 Gew.-% bis 40 zu 60 Gew.-% beträgt, z.B. mittels dip coating, spin coating oder spray coating, auf die Bereiche des Implantates aufgebracht wird, die hydrophilisiert wurden,
- die so entstandene Schicht getrocknet wird, und
- anschließend die Kunstoffimplantatoberfläche soweit erhitzt wird, dass der geschmolzene Kunststoff in Poren der entstandenen bzw. aufgetragenen Schicht eindringt, insbesondere in Nanoporen.

Dabei wird das Siliziumdioxidsol mit dem dispergierten Hydroxylapatit dadurch hergestellt, dass Natriumhydrogenphosphat und Caciumchlorid zur Fällung von HA genutzt wird, dass, z.B. durch Spülen und Abfiltern, die Konzentration der Natrium und Chlor Ionen erniedrigt wird, wobei die Ionenkonzentration bevorzugt auf weniger als 0.1% der Anfangskonzentration verringert wird, dass, z.B. durch Hinzufügen und Abfiltern von Ethanol, der Wassergehalt reduziert wird, wobei der Wassergehalt (danach) bevorzugt weniger als 1% vom Gesamtlösungsmittel beträgt, dass durch die Hydrolyse von Tetraethylorthosilikat (TEOS) bei der bevorzugten Verwendung einer organischen Säure, besonders bevorzugt Essigsäure, als Katalysator ein Kieselsäuresol erzeugt wird, das mit der Hydroxylapatitsuspension vermischt wird.

In dem zweiten erfindungsgemäßen Verfahren wird also, um das Kunststoffimplantat beschichten zu können, die Oberfläche z.B. durch ein Sauerstoffplasma hydrophilisiert. Hierzu kann ein Normaldruck Plasmajet (z.B. mit Plasma Beam, diener electronic GmbH) oder Niederdruckplasma (z.B. mit Nano PCCE, diener electronic GmbH) genutzt werden. Für die Beschichtung wird ein Siliziumdioxidsol hergestellt, in das kristallines HA dispergiert wird. Die Feststoffkonzentration (HA und SiO₂ des Sols soll im Bereich von 0.2 bis 10 Gew.-% liegen. Das Verhältnis von HA zu SiO₂ wird im Bereich von 90 zu 10 Gew.% bis 40 zu 60 Gew.% festgelegt. Z.B. mittels dip coating, spin coating oder spray coating können nun die Bereiche des Implantates beschichtet werden, an die Knochen anwachsen soll. Die anderen Bereiche des Implantates können zum Beispiel abgedeckt werden. Die Schicht wird getrocknet. Der Vorgang kann gegebenenfalls mehrfach (z.B. zweimal, dreimal, viermal oder fünfmal) wiederholt werden, um eine entsprechende Schichtdicke zu erreichen. Der innere Aufbau der Schicht bis in den Nanometerbereich ist nun vergleichbar mit dem oben beschriebenen Knochenersatzmaterial bzw. entspricht dem Knochenersatzmaterial nach EP 1624904B1.

Optional kann die Schicht aus Siliziumdioxid und HA vor dem Erhitzen der Kunststoffoberfläche durch ein Sauerstoffplasma aktiviert werden.

Anschließend wird die Kunstoffimplantatoberfläche soweit erhitzt, dass der Kunststoff in die Poren, insbesondere. Nanoporen, der entstandenen Schicht eindringt. Dies kann z.B. mit einem heißen Luftstrom (z.B. ca. 300-400°C, bevorzugt ca. 350-380°C für ca. 2-30 Sekunden, bevorzugt 5-20 oder 10-15 Sekunden) erfolgen. Die Schicht versinkt praktisch im Kunststoff soweit, dass nur der obere Teil herausschaut.

Nach dem Abkühlen kann die Oberfläche noch einmal mit Sauerplasma behandelt werden, um gegebenenfalls organische Reste, die aus dem Sol stammen können, zu entfernen. Wenn nach dem Beschichten und vor dem Erhitzen bereits eine Aktivierung mit Sauerstoffplasma stattgefunden hat, ist eine zweite Behandlung nicht nötig. Abhängig z.B. von Lagerung und Einsatzzweck kann jedoch auch auf beide Behandlungen mit Sauerstoffplasma nach dem Beschichten des Implantats verzichtet werden.

Wenn gewünscht, kann auf die erfindungsgemäße, bereits teilweise eingeschmolzene Schicht des Knochenersatzmaterials eine weitere Schicht Knochenersatzmaterial aufgebracht werden. Dazu kann man ein Siliziumdioxidsol, in das kristallines Hydroxylapatit dispergiert ist, wie oben detailliert beschrieben, z.B. mittels dip coating, spin coating oder spray coating, auf die Bereiche des Implantates aufbringen, auf denen eine dickere Schicht Knochenersatzmaterial, die aus dem Kunststoff herausragt, gewünscht ist. Dies kann z.B. dann sinnvoll sein, wenn ein Versinken der Schicht aufgrund der Form des Implantats nicht gleichmäßig ist.

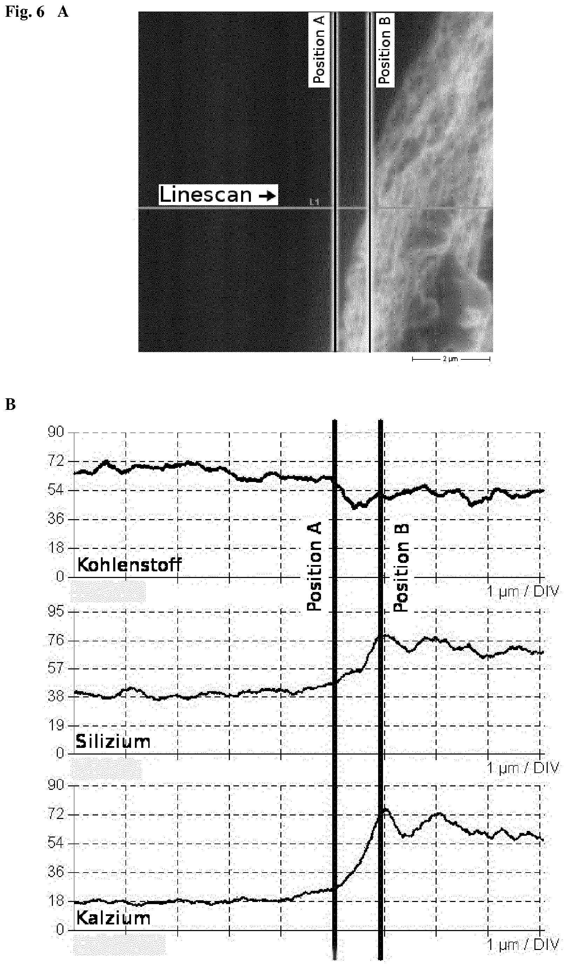

Auch ein mit diesem zweiten Verfahren herstellbares oder hergestelltes Kunststoffimplantat, insbesondere ein nicht rotationssymmetrisches Kunststoffimplantat, ist Gegenstand der vorliegenden Erfindung. Dieses unterscheidet sich von mit dem ersten Verfahren hergestellten erfindungsgemäß beschichteten Implantaten dadurch, dass nicht Granulatkörner des Knochenersatzmaterials teilweise in den Kunststoff eingebettet sind, sondern eine homogene Schicht des Knochenersatzmaterials teilweise in den Kunststoff eingebettet ist.. Beschrieben ist also ein Kunststoffimplantat, bevorzugt aus PEK, PEKK oder insbesondere PEEK, bei dem in den Bereichen, in denen Knochen an das Implantat anwachsen soll, in der Oberflächenschicht eine homogene Schicht eines Knochenersatzmaterials im Kunststoff eingebettet ist, wobei das Knochenersatzmaterial aus der Oberfläche herausragt. Homogen bezieht sich dabei auf die gleichmäßige Verteilung des Knochenersatzmaterials in Richtung der Kunststoffoberfläche. In dazu vertikaler Richtung liegt, wie z.B. in Fig. 6B gezeigt, keine Homogenität vor, sondern zum Implantat hin wird der Kunststoffanteil größer. Ein solches Kunststoffimplantat ist z.B. mit dem zweiten erfindungsgemäßen Verfahren herstellbar. Dabei liegt die Porosität des Knochenersatzmaterials bevorzugt im Bereich von 20 bis 80%, d.h., das Knochenersatzmateral ist hochporös, und/oder die mittlere Porengröße (des Knochenersatzmaterials) liegt bevorzugt im Bereich von 10 bis 100 nm. Die Oberflächenschicht des Kunststoffes weist bevorzugt eine Stärke von 1µm bis 100µm auf und/oder das Knochenersatzmaterial ragt bevorzugt im Bereich von 0,01 µm - 50 µm, mehr bevorzugt im Bereich von 0.1 bis 30µm oder im Bereich von 1 bis 20 µm aus dem Kunststoff heraus. Es wird auch ein mit dem oben beschriebenen ersten beschriebenen Verfahren herstellbares oder hergestelltes Kunststoffimplantat, insbesondere ein rotationssymmetrisches Kunststoffimplantat.

Selbstverständlich kann mit den erfindungsgemäßen Verfahren auch ein Kunstoffimplantat auf seiner gesamten Oberfläche beschichtet werden, wenn es z.B. an der ganzen Oberfläche nach dem Implantieren Kontakt zu Knochen haben soll, wie etwa bei einem Cage zur Fusionierung von Wirbeln.

Durch das Durchdringen der Nanoporen mit dem Kunststoff ist die Oberflächenschicht mechanisch sehr stabil. Für die Gewebeinteraktion muss von dem Knochersatzmaterial keine extra Schicht auf dem Kunststoff Silicagel/HA Verbund existieren, d.h., es ist ausreichend, wenn das Knochenersatzmaterial sich an der Oberfläche befindet, es muss nicht unbedingt herausragen. Entsprechend der Wirksamkeit des Materials ist eine zusätzliche Schicht von einigen Mikrometern aber von Vorteil. Durch die neue nanostrukturierte Oberfläche des PEEK wird ein vollständiger Abbau der untersten Knochenersatzmaterialschicht durch knochenabbauenden Zellen verhindert, da diese nicht in diese Struktur eindringen können. Es bleibt letztendlich eine dünne Biomaterialschicht auf der PEEK-Oberfläche erhalten. Beschrieben ist auch die Verwendung eines erfindungsgemäßen Kunststoffimplantats zum Implantieren, insbesondere als Implantat mit Implantat-Knochen-Kontakt, bevorzugt in der Unfallchirurgie, der Orthopädie und/oder insbesondere in der Wirbelsäulenchirurgie. Das Kunststoffimplantat kann z.B. ein Cage, ein Implantat zur spinalen Fusion, sein. Auch erfindungsgemäß beschichtete Zahnimplantate können verwendet werden.

Tierexperimente zeigen eine Verbesserung des Knochen-Implantat-Kontakts um bis zu 15% für erfindungsgemäße Implantate gegenüber entsprechenden konventionellen, nicht beschichteten Kunstoffimplantaten und belegen damit die Vorteile der Erfindung.

### Legende

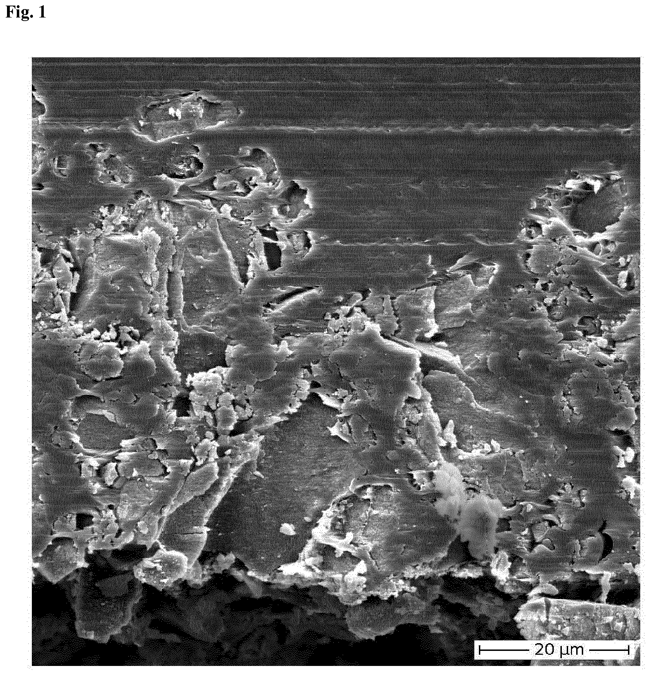

**Fig. 1** zeigt eine zum Teil in PEEK versunkene Schicht des nach Beispiel 1 hergestellten Knochenersatzmaterials. Skala: 20 µm.

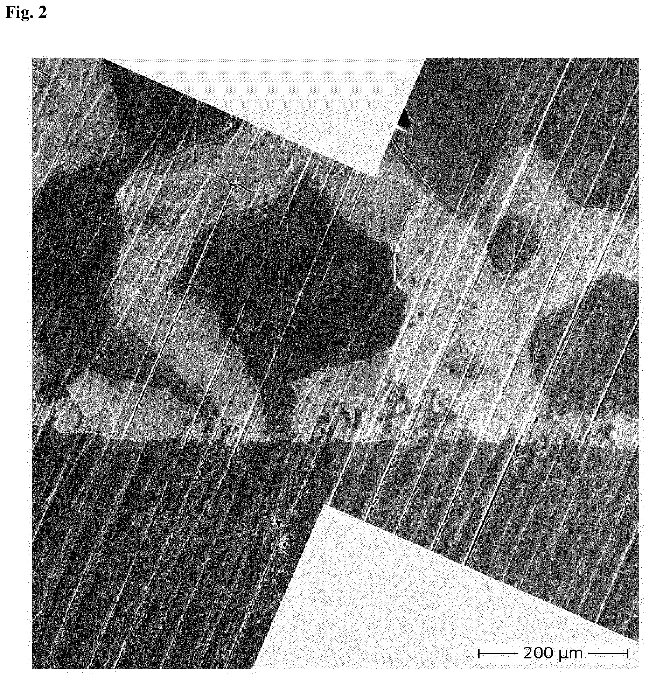

**Fig. 2** zeigt eine rasterelektronenmikroskopische Aufnahme eines beschichteten PEEK-Formkörpers nach 6 Wochen *in vivo.* Mit der rasterelektronenmikroskopischen Aufnahme läßt sich der elementspezifische Verbund durch EDX (Energiedispersive Röntgenspektroskopie)-Messungen bestätigen. Skala: 200 µm.

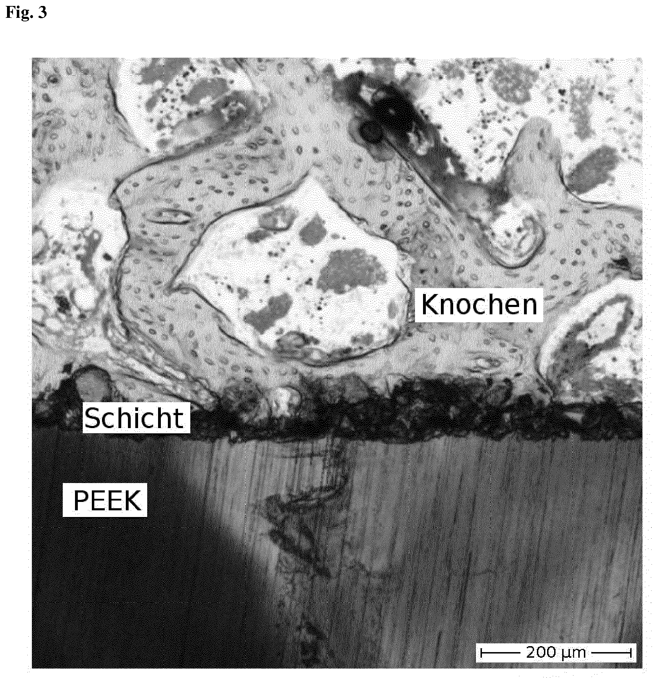

**Fig. 3** zeigt das gleiche Präparat wie Fig. 2, nur als auflichtmikroskopische Aufnahme des histologischen Dünnschliffes. Die Auflichtaufnahme zeigt deutlich die Strukturänderung des Polymers durch den Beschichtungsvorgang und das an der Beschichtung verankerte Knochengewebe. Skala: 200 µm.

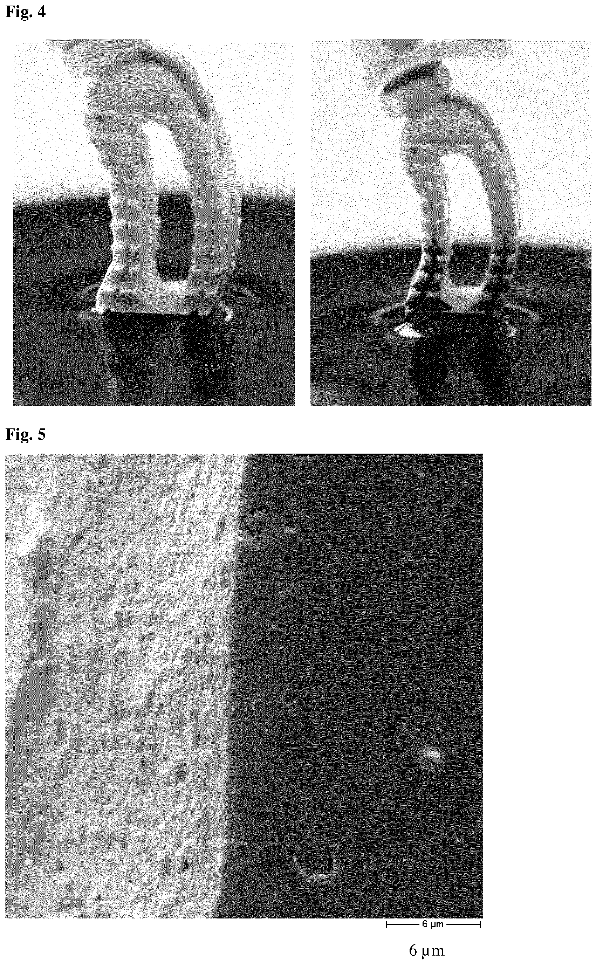

**Fig. 4** zeigt je zwei PEEK-Cages, welche mit der unteren Spitze in schwarz eingefärbtes Wasser getaucht wurden. Auf der linken Seite (A) findet keine Benetzung statt, da das Implantat eine hydrophobe Oberfläche hat. Auf der rechten Seite (B) findet eine starke Benetzung statt, nachdem das Implantat durch eine Plasmabehandlung eine hydrophile Oberfläche bekommen hat.
**Fig. 5** ist eine Darstellung einer nach Beispiel 3 durch zweifaches Einsprühen aufgebrachten Schicht durch eine rasterelektronische Aufnahme, auf der rechts PEEK und in der Mitte die ewa 5 µm dicke eingeschmolzene Beschichtung mit Knochenersatzmaterial zu sehen ist (Skala: 6 µm).
**Fig. 6** zeigt eine rasterelektonenmikroskopische Aufnahme eines Schnitts eines erfindungsgemäßen Implantats (A, Skala 2 µm) und eine Elementanalyse entlang der in Fig. 6A eingezeichneten horizontalen Linie (Linescan) (B). Der durch vertikale Linien (Position A und B) markierte Bereich ist in Fig. 6A und Fig. 6B identisch und stellt den Bereich der aufgetragenen Schicht dar. Fig. 6B zeigt von oben nach unten die Anteile von Kohlenstoff, Silizium und Kalzium. Dabei zeigt sich, dass die Schicht einen Gradienten aufweist. Die Schicht ist im unteren Bereich (in Fig. 6A und B links) stärker vom PEEK (Kohlenstoff) durchdrungen als im oberen Bereich (in Fig. 6A und B rechts).

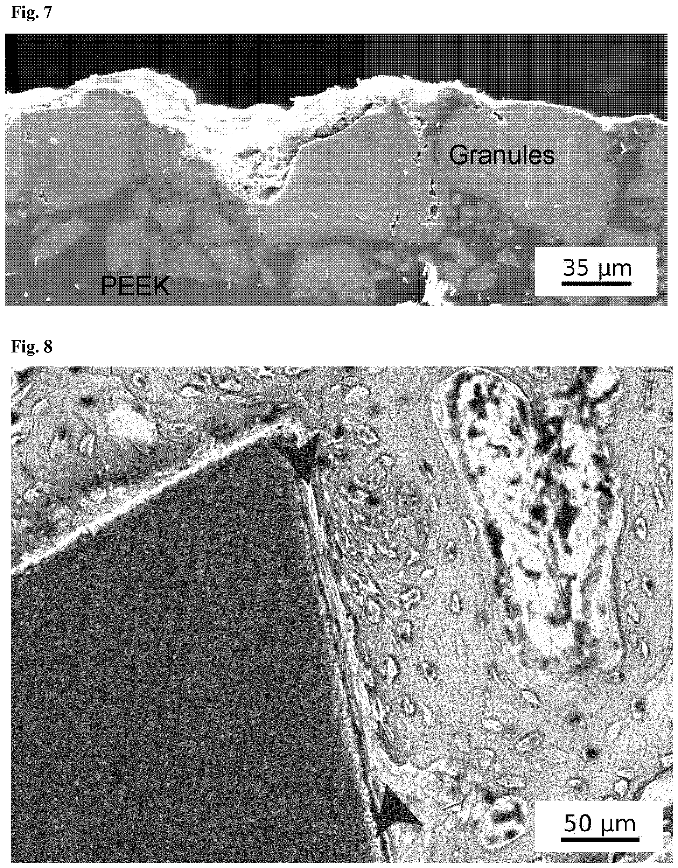

**Fig. 7** zeigt eine zum Teil in PEEK versunkene Schicht des nach Beispiel 1 hergestellten Knochenersatzmaterials. Skala 35 µm.
**Fig. 8** zeigt eine typische lichtmikroskopische Aufnahme eines histologischen Präparates aus dem in vivo-Experiment in Beispiel 2 aus der Kontrollgruppe nach 2 Wochen. Dabei zeigt sich das Problem der schlechten Zelladhäsion anhand von Bindegewebsschichten zwischen Knochengewebe und der PEEK-Implantatoberfläche (Pfeile).

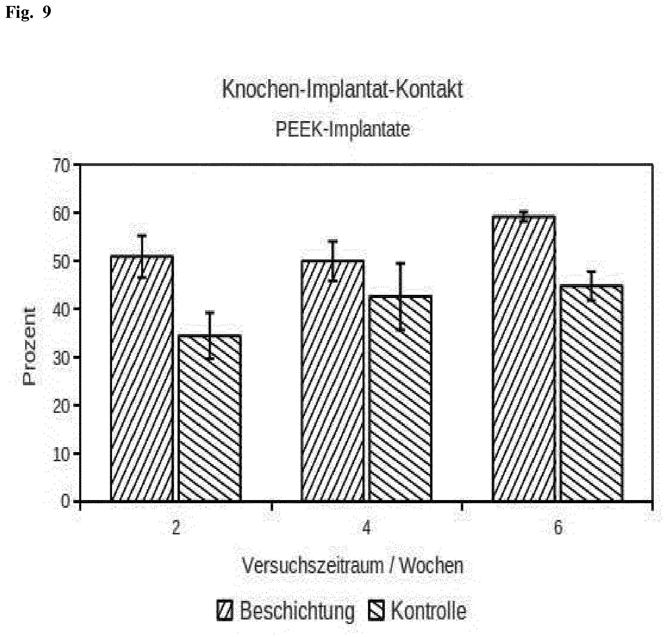

**Fig. 9** zeigt Messwerte des Knochen-Implantat-Kontaktes aus dem *in vivo* Experiment durch die erfindungsgemäße Beschichtung in Beispiel 2. Nach 2 Wochen Standzeit wurde eine Verbesserung des Knochen-Implantat-Kontakts durch die Beschichtung von etwa 15% gemessen, nach 4 Wochen eine von etwa 10% und nach 6 Wochen etwa 15%. Die Messwerte wurden mit der semiautomatischen Software Axio Vision 4.8 (Zeiss) gemessen.

### Beispiel 1

### Anwendung einer NanoBone® S39-Pulverbeschichtung auf rotationssymetrischen PEEK-Formkörpern.

NanoBone® S39-Granulat (Artoss GmbH, Rostock, Knochenersatzmaterial hergestellt nach EP 1624904 B1) wird zu einem feinen Pulver gemahlen (Korngröße 5-100µm). Der zu beschichtende PEEK-Körper wird in eine extra für diesen Körper vorgesehene zylindrische Aussparung (Parallelstellung der Rotationsachsen vom PEEK-Körper und der Aussparung) in einer Edelstahlform eingelassen. Der Freiraum zwischen dem zu beschichtenden Körper und der Metallform wird mit dem gemahlenen Granulat gefüllt und durch leichten Druck (z.B ca. 0,1 bis ca. 5 MPa) in Richtung der Rotationsachse des PEEK-Körpers verdichtet. Die Edelstahlform (mit PEEK-Körper und gemahlenem Granulat) wird dann in eine Induktionsspule (TIG 30/100, HÜTTINGER Elektronik GmbH) eingeführt. Durch den Induktionsprozess kann dem PEEK-Körper sehr schnell Wärme zugeführt werden (z.B. 380°C bis 400°C, gemessen an der Oberfläche der Form, für 5 Sekunden, was nur zu einem Aufschmelzen der PEEK-Oberfläche führt. Das geschmolzene PEEK passt sich der nanoporösen Oberfläche des NanoBone® an und stellt dabei eine feste Verbindung her. Abbildung 1 und 7 zeigen die versunkene Biomaterialschicht im PEEK.

### Beispiel 2

Die nach Beispiel 1 beschichteten Formkörper wurden in einem *in vivo* Experiment an weißen New Zealand Kaninchen mit 2, 4 und 6 Wochen Standzeit mit jeweils 6 Implantaten in der Kontrollgruppe (reine PEEK-Oberfläche) und 6 Implantaten in der Beschichtungsgruppe getestet. Die Implantate wurden lateral in den distalen Femurknochen eingesetzt. Entsprechende Versuche dienen im Stand der Technik häufig zum Testen von Zahnimplantaten. Nach der Euthanasie wurden die Implantate durch die Dünnschlifftechnik aufbereitet und mit Toluidinblau angefärbt, um eine histologische und histomorphometrische Auswertung durchführen zu können.

Die neue nanostrukturierte Oberfläche des PEEK verhindert ein Eindringen von knochenabbauenden Zellen, allein durch die neue Größenordnung der Struktur in der Oberfläche. Auch die makroskopische Rauheit, welche durch den Beschichtungsvorgang erzeugt wird, kann eine Rolle spielen.

Das hat zur Folge, dass keine Zell-PEEK Grenzfläche mehr vorliegt sondern eine Zell-NanoBone® Grenzfläche, wobei das NanoBone® mit dem PEEK fest verbunden ist. Fig. 2 zeigt eine rasterelektronenmikroskopische Aufnahme eines beschichteten PEEK-Formkörpers nach 6 Wochen *in vivo.* Mit der rasterelektronenmikroskopischen Aufnahme läßt sich der elementspezifische Verbund durch EDX Messungen bestätigen. Fig. 3 zeigt das gleiche Präparat wie Fig. 2, nur als auflichtmikroskopische Aufnahme des histologischen Dünnschliffes. Die Auflichtaufnahme zeigt deutlich die Strukturänderung des Polymers durch den Beschichtungsvorgang und das an der Beschichtung verankerte Knochengewebe.

Fig. 8 zeigt eine typische lichtmikroskopische Aufnahme eines histologischen Präparates der Kontrollgruppe nach 2 Wochen. Dabei zeigt sich das Problem der schlechten Zelladhäsion anhand von Bindegewebsschichten zwischen Knochengewebe und der PEEK-Implantatoberfläche (Pfeile).

Zusätzlich zeigt das *in vivo* Experiment eine Steigerung des Knochen-Implantat-Kontaktes um ca. 10-15% (siehe Fig. 9, gemessen mit der semiautomatischen Software Axio Vision 4.8 (Zeiss)).

### Beispiel 3

Ein Siliziumdioxidsol mit dem dispergierten Hydroxylapatit wird dadurch hergestellt, dass Natriumhydrogenphosphat und Calciumchlorid zur Fällung von HA genutzt wird, dass durch Spülen und Abfiltern die Konzentration der Natrium und Chlor Ionen erniedrigt wird, wobei die Ionenkonzentration auf ca. 0.1% der Anfangskonzentration verringert wird, dass durch Hinzufügen und Abfiltern von Ethanol der Wassergehalt reduziert wird, wobei der Wassergehalt ca. 1% vom Gesamtlösungsmittel beträgt. Durch eine Hydrolyse von Tetraethylorthosilikat (TEOS) bei der Verwendung von Essigsäure als Katalysator wird ein Kieselsäuresol erzeugt. Nun wird Ethanol, das Sol und die HA Suspension zu solchen Teilen vermischt, dass ein Feststoffanteil (SiO2 und HA) von 1 % und ein Massenverhältnis von HA zu SiO2 von 76 zu 24 entsteht.

Ein PEEK Implantat, wie es in der Wirbelsäulenchirurgie verwendet wird (Cage), wird im Niederdrucksauerstoffplasma für 60 Sekunden behandelt, um eine benetzende Oberfläche zu erzeugen. In Abbildung 4 wurden je zwei PEEK-Cages mit der unteren Spitze in schwarz eingefärbtes Wasser getaucht. Auf der linken Seite findet keine Benetzung statt, da das Implantat eine hydrophobe Oberfläche hat. Auf der rechten Seite findet eine starke Benetzung statt, nachdem das Implantat durch eine Plasmabehandlung eine hydrophile Oberfläche bekommen hat.

Anschließend wird das PEEK Implantat, während es rotiert, mit dem hergestellten Sol eine Sekunde eingesprüht und im Luftstrom getrocknet. Das ganze wird, falls für die Schichtdicke gewünscht, mehrfach, z.B. zweimal oder dreimal wiederholt.

Anschließend wird das rotierende Implantat in einen Luftstrom von 350°C gebracht, so dass die Oberfläche in den flüssigen Zustand übergeht und die Schicht "versinkt". Darstellung der aufgebrachten Schicht in Abbildung 5 durch eine rasterelektronische Aufnahme. In Abbildung 6 wurde eine Elementanalyse entlang der horizontalen Linie (Linescan) durchgeführt. Der durch vertikale Linien (Position A und B) markierte Bereich ist in der linken Abbildung und dem Diagramm auf der rechten Seite identisch und stellt den Bereich der aufgetragenen Schicht dar. Das Diagramm auf der rechten Seite zeigt von oben nach unten die Anteile von Kohlenstoff, Silizium und Kalzium. Dabei zeigt sich, dass die Schicht einen Gradienten aufweist. Die Schicht ist im unteren Bereich (im Diagramm links) stärker vom PEEK (Kohlenstoff) durchdrungen als im oberen Bereich (im Diagramm rechts).

Die Beseitigung organischer Reste aus der Oberfläche der Schicht geschieht durch eine abschließende Sauerstoffplasmabehandlung.

## Patentansprüche

1. Verfahren zur Herstellung eines Kunststoffimplantats wobei das Kunststoffimplantat **dadurch gekennzeichnet ist, dass** in den Bereichen, in denen Knochen an das Implantat anwachsen soll, in der Oberflächenschicht ein Knochenersatzmaterial im Kunststoff eingebettet ist, wobei das Knochenersatzmaterial aus der Oberfläche herausragt und die Porosität des Knochenersatzmaterials im Bereich von 20 bis 80% liegt, wobei das Verfahren **dadurch gekennzeichnet ist, dass** eine Form zur Herstellung des Implantates an den Stellen, an denen das Implantat Knochenkontakt haben soll, mit einem wässrigen Schlicker aus einem hochporösem Knochenersatzmaterial beschichtet wird, die Schicht getrocknet und anschließend der plastifizierte Kunststoff in die Form gegeben wird, wobei der Kunststoff Polyaryl-Polyetherketon (PEK) ist, und wobei der Schlicker aus Wasser und einem Granulat von kristallinem Hydroxylapatit (HA), eingebettet in einer amorphen porösen Matrix aus Siliziumdioxid, besteht, wobei die Granulatgröße bevorzugt im Bereich von 1 bis 50 µm liegt.

2. Verfahren zur Herstellung eines Kunststoffimplantats nach Anspruch 1, **dadurch gekennzeichnet, dass** man die Oberfläche des Kunststoffs zum Schmelzen bringt.

3. Verfahren zur Herstellung eines Kunststoffimplantats wobei das Kunststoffimplantat **dadurch gekennzeichnet ist, dass** in den Bereichen, in denen Knochen an das Implantat anwachsen soll, in der Oberflächenschicht ein Knochenersatzmaterial im Kunststoff eingebettet ist, wobei das Knochenersatzmaterial aus der Oberfläche herausragt und die Porosität des Knochenersatzmaterials im Bereich von 20 bis 80% liegt, wobei das Verfahren, **dadurch gekennzeichnet ist,**
**dass** die Bereiche des Implantates, an denen Knochen anwachsen soll hydrophilisiert werden,
**dass** ein Siliziumdioxidsol, in dem kristallines Hydroxylapatit (HA) dispergiert ist, wobei die Feststoffkonzentration (HA und SiO₂) des Sols im Bereich von 0.2 bis 10 Gew.-% liegt und das Verhältnis von HA zu SiO₂ im Bereich von 90 zu 10 Gew.-% bis 40 zu 60 Gew.-% beträgt, auf die Bereiche des Implantates aufgebracht wird, die hydrophilisiert wurden,
**dass** die so entstandene Schicht getrocknet wird, und
**dass** anschließend die Kunstoffimplantatoberfläche soweit erhitzt wird, dass der Kunststoff in Nanoporen der entstandenen Schicht eindringt, wobei der Kunststoff Polyaryl-Polyetherketon (PEK) ist.

4. Verfahren zur Herstellung eines Kunststoffimplantats nach Anspruch 3, **dadurch gekennzeichnet, dass** das Siliziumdioxidsol mit dem dispergierten Hydroxylapatit dadurch hergestellt wird, dass Natriumhydrogenphosphat und Calciumchlorid zur Fällung von HA genutzt wird, dass die Konzentration der Natrium- und Chlorionen erniedrigt wird, dass der Wassergehalt auf weniger als 1% vom Gesamtlösungsmittel reduziert wird, dass durch die Hydrolyse von Tetraethylorthosilikat (TEOS) ein Kieselsäuresol erzeugt wird, das mit der Hydroxylapatitsuspension vermischt wird.

5. Verfahren zur Herstellung eines Kunststoffimplantats nach Anspruch 4, **dadurch gekennzeichnet, dass** die Ionenkonzentration auf weniger als 0.1 % der Anfangskonzentration verringert wird.

6. Verfahren zur Herstellung eines Kunststoffimplantats nach einem der Ansprüche 4 oder 5, **dadurch gekennzeichnet, dass** das Kieselsäuresol durch die Hydrolyse von Tetraethylorthosilikat (TEOS) bei der Verwendung einer organischen Säure als Katalysator erzeugt wird.

7. Verfahren zur Herstellung eines Kunststoffimplantats nach einem der Ansprüche 4 bis 6, **dadurch gekennzeichnet, dass** die organische Säure Essigsäure ist.

8. Verfahren zur Herstellung eines Kunststoffimplantats nach einem der Ansprüche 3 oder 4, **dadurch gekennzeichnet, dass** die Schicht aus Siliziumdioxid und HA vor oder nach dem Erhitzen der Kunststoffoberfläche durch ein Sauerstoffplasma aktiviert wird.

## Claims

1. Method for producing a plastic implant, wherein the plastic implant is **characterized in that** in the surface layer a bone substitute material is embedded in the plastic in the areas in which bone shall grow onto the implant, wherein the bone substitute material protrudes from the surface and the porosity of the bone substitute material is in the range of 20 to 80%, wherein the method is **characterized in that** a mold for the manufacture of the implant is coated with an aqueous slurry of a highly porous bone substitute material in the areas in which the implant shall have contact with the bone, the layer is dried and subsequently the plasticized plastic is introduced into the mold, wherein the plastic is polyaryl polyether ketone (PEK), and wherein the slurry consists of water and a granulate of crystalline hydroxyapatite (HA), embedded in an amorphous porous matrix of silicon dioxide, wherein the size of the granulate preferably is in the range of 1 to 50 µm.

2. Method for producing a plastic implant according to claim 1, wherein the surface of the plastic is caused to melt.

3. Method for producing a plastic implant, wherein the plastic implant is **characterized in that** in the surface layer a bone substitute material is embedded in the plastic in the areas in which the bone shall grow onto the implant, wherein the bone substitute material protrudes from the surface, and the porosity of the bone substitute material is in the range of 20 to 80%, wherein the method is **characterized in that** the areas in which the bone shall grow onto the implant are hydrophilized, a silicon dioxide sol is applied to the hydrophilized areas of the implant in which crystalline hydroxyapatite (HA) is dispersed, wherein the solid matter concentration (HA and SiO₂) of the sol is in the range of 0.2 to 10% by weight, and the ratio of HA to SiO₂ is in the range of 90 to 10% by weight to 40 to 60% by weight, the resulting layer is dried, and the plastic implant surface is subsequently heated to an extent that the plastic penetrates into nanopores of the layer so formed, wherein the plastic is polyaryl polyether ketone (PEK) .

4. Method for producing a plastic implant according to claim 3, **characterized in that** the silicon dioxide sol with the dispersed hydroxyapatite is manufactured by using sodium hydrogen phosphate and calcium chloride for precipitation of HA, the concentration of sodium and chlorine ions is decreased, the water content is reduced to less than 1% of the total solvent, a silica sol is generated by the hydrolysis of tetraethyl orthosilicate (TEOS), which is mixed with the hydroxyapatite suspension.

5. Method for producing a plastic implant according to claim 4, **characterized in that** the ion concentration is decreased to less than 0.1% of the initial concentration.

6. Method for producing a plastic implant according to any one of claims 4 or 5, **characterized in that** the silica sol is produced by the hydrolysis of tetraethyl orthosilicate (TEOS) using an organic acid as a catalyst.

7. Method for producing the plastic implant according to any one of claims 4 to 6, **characterized in that** the organic acid is acetic acid.

8. Method for producing the plastic implant according to any one of claims 3 or 4, **characterized in that** the layer of silicon dioxide and HA is activated by an oxygen plasma before or after heating of the plastic surface.

## Revendications

1. Procédé de réalisation d'un implant en matière plastique, selon lequel l'implant en matière plastique est **caractérisé par le fait que** dans les zones où l'os doit pousser sur l'implant, un matériau de substitution osseuse est encastré dans la couche superficielle de la matière plastique, le matériau de substitution osseuse dépassant de la surface, et la porosité du matériau de substitution osseuse se situant dans la plage allant de 20 à 80 %, le procédé étant **caractérisé en ce que** pour la réalisation de l'implant, un moule est recouvert d'une barbotine aqueuse, obtenue à partir d'un matériau de substitution osseuse hautement poreux, aux endroits où l'implant doit être en contact avec l'os, que la couche est séchée et qu'ensuite la matière plastique plastifiée est placée dans le moule, la matière plastique étant du polyaryléthercétone (PEK), et la barbotine se composant d'eau et de granulés d'hydroxyapatite (HA) cristalline, encastrés dans une matrice poreuse amorphe de silice, la taille des granulés se situant de préférence dans la plage allant de 1 à 50 µm.

2. Procédé de réalisation d'un implant en matière plastique selon la revendication 1, **caractérisé en ce que** la surface de la matière plastique est mise en fusion.

3. Procédé de réalisation d'un implant en matière plastique, selon lequel l'implant en matière plastique est **caractérisé par le fait que** dans les zones où l'os doit pousser sur l'implant, un matériau de substitution osseuse est encastré dans la couche superficielle de la matière plastique, le matériau de substitution osseuse dépassant de la surface, et la porosité du matériau de substitution osseuse se situant dans la plage allant de 20 à 80 %, le procédé étant **caractérisé en ce que**
les zones de l'implant où l'os doit pousser sont hydrophilisées,
**en ce qu'**un sol de dioxyde de silicium, dans lequel est dispersé de l'hydroxyapatite (HA) cristalline - sachant que la concentration en matières solides (HA et SiO₂) du sol se situe dans la plage allant de 0,2 à 10 % en poids, et le rapport de HA à SiO₂ se situe dans la plage allant de 90 à 10 % en poids jusqu'à 40 à 60 % en poids - est appliqué sur les zones de l'implant qui ont été hydrophilisées,
**en ce que** la couche ainsi formée est séchée, et
**en ce qu'**ensuite la surface de l'implant en matière plastique est chauffée de manière telle que la matière plastique pénètre dans des nanopores de la couche formée, sachant que la matière plastique est du polyaryléthercétone (PEK).

4. Procédé de réalisation d'un implant en matière plastique selon la revendication 3, **caractérisé en ce que** le sol de dioxyde de silicium avec l'hydroxyapatite dispersée est fabriqué par le fait que l'on utilise de l'hydrogénophosphate de sodium et du chlorure de calcium pour la précipitation de HA, que l'on abaisse la concentration des ions de sodium et de chlore, que l'on ramène la teneur en eau à moins de 1 % du solvant total, que l'on produit un sol de silice par hydrolyse d'orthosilicate de tétraéthyle (TEOS) qui est mélangé avec la suspension d'hydroxyapatite.

5. Procédé de réalisation d'un implant en matière plastique selon la revendication 4, **caractérisé en ce que** la concentration d'ions est ramenée à moins de 0,1 % de la concentration initiale

6. Procédé de réalisation d'un implant en matière plastique selon une des revendications 4 ou 5, **caractérisé en ce que** le sol de silice est produit par hydrolyse d'orthosilicate de tétraéthyle (TEOS), avec utilisation d'un acide organique en tant que catalyseur.

7. Procédé de réalisation d'un implant en matière plastique selon une des revendications 4 à 6, **caractérisé en ce que** l'acide organique est de l'acide acétique.

8. Procédé de réalisation d'un implant en matière plastique selon une des revendications 3 ou4, **caractérisé en ce que** la couche de dioxyde de silicium et de HA est activée par un plasma d'oxygène, avant ou après le chauffage de la surface en matière plastique.
